# EUROPEAN PATENT APPLICATION

(11) **EP 4 726 093 A1**
(43) Date of publication of application: **15.04.2026**
(21) Application number: 24205790.9
(22) Date of filing: 10.10.2024
(51) Int. Cl.: D04H 1/72, A61L 15/48, B41M 1/40, B41M 3/00, D06M 15/37, D06M 23/16

(54) **PRINTING HYDROPHILIC AREAS ON NONWOVEN MATERIALS**

(71) Applicant: Fibertex Personal Care A/S, 9220 Aalborg (DK)
(72) Inventor: JACOBSEN, Jesper B., 9270 Klarup (DK)
(74) Representative: Lorenz Seidler Gossel Part. mbB

(57) **Abstract**

The invention relates to nonwoven materials for the hygiene field, and more particular to nonwoven materials having hydrophilic properties imparted over at least part of its surface, and method for making them. The method comprises depositing a solution of a surfactant on the surface of the nonwoven material by printing.

## Description

### Field of the invention

The invention relates to nonwoven materials for the hygiene field, and more particular to nonwoven materials having hydrophilic properties imparted in a pattern, and method for making them.

### Background of the invention

In absorbent hygiene products (AHP), it is a common practice to implement nonwoven hydrophilic layers into the product in order to let and guide body fluids into the absorbent core of the product. The various layers in the AHP are usually made from polyolefins nonwoven materials, which are naturally hydrophobic. Hence, they need a treatment to become hydrophilic. This can be done by coating them with various surfactants that are available by multiple suppliers in the industry.

The surfactant is usually mixed with a liquid carrier down to an active concentration level between 5-20%. Application of the mixed surfactant onto the substrate (nonwoven) is usually done by kiss roll equipment.

When the materials is being made and moving fast in the machine direction, these coating devices can either apply the surfactant continuously over the full width of the material or in partial zones, which are then machine-directional stripes in the product. The add on level, also known as oil pick up (OPU), is determined by the surfactant concentration, web speed and the rotation speed of the kiss roll and will be the same in all areas where surfactant is applied.

For some applications, however, it could be advantageous to make the materials hydrophilic in ways that can be individually designed beyond full width or machine-directional stripe design with constant OPU, to impart certain performance characteristic. The present invention aims to provide solutions in this respect.

### Summary of the invention

Against this background, the invention relates to a method for producing a nonwoven material, whose surface is at least in part treated for increased hydrophilicity by depositing a solution of a surfactant on the surface, wherein the solution of surfactant is applied to the surface by printing. The invention further relates to a nonwoven material made by such method. In such nonwoven material, as a result of the method, the surface of the nonwoven material is at least in part treated for increased hydrophilicity by having deposited a surfactant thereon by printing.

### Detailed description of the invention

The surface of the nonwoven as used herein is one of the two opposing surfaces of the nonwoven.

Printing is used in the prior art for creating a visual appearance on the nonwoven substrate. Reference can be made to WO 2023/166106 A1 and many other documents. The present invention uses printing for a different purpose, however, namely the imparting of hydrophilic properties in a pattern. This leads to advantages over kiss roll application, which was used for imparting hydrophilic properties in the prior art.

The surfactant is typically selected from cationic surfactants such as quaternary ammonium compounds (quats) or imidazolium derivatives, anionic surfactants such as sodium lauryl sulphate (SLS) and alkylbenzene sulfonates, nonionic surfactants such as ethoxylated alcohols and alkylphenol ethoxylates, and silicone-based surfactants such as silicone polyesters or cationic silicone surfactants.

Preferably, the printing as used in the method of the present invention is flexographic printing. In flexographic printing, the solution is transferred from an anilox roll to a print roll and then to the surface of the nonwoven material, which translates in machine direction through the nip between the print roll and an impression drum. The print roll usually has a cylindrical print sleeve mounted on a core cylinder. The surface comprises a mirrored master of the required image as a positive 3D relief. Ink is transferred from an ink reservoir to the print roll. The anilox roll transfers solution from a reservoir to the print roll and has surface cells for dosing the solution. Doctor blades scrape off excessive solution from outside the cells.

In general, the method is applicable to both, full width homogeneous application of surfactant to the surface and zone-wise application in stripes or patterns.

A main advantage of the present invention is the more precise control and option to vary application amounts and that the hydrophilic areas do not have to be continuous or in the form of straight machine-directional stripes, but can have any odd shape, where any odd shape can additionally have any defined application amount.

The application of the solution of surfactant to the surface of the nonwoven material is such that at least two different amounts of the solution are applied to at least two different zones of the surface. One of the amounts may be zero. In embodiments, at least one of the zones may have a shape other than machine-directional stripes with a constant width and linear boundary, or the at least two different amounts of the solution that are applied to the at least two different zones of the surface may be at least two different non-zero amounts, or both.

The relief area on a flexographic print roll can be solid for a maximum solution transfer amount, or can be made with various screenings to make the area more or less concentrated. Screenings can be created by having tiny dots of relief peaks as surface and areas where the peaks are removed. A 50% coverage would be an equal mix of peaks and open area, whereas a 100% screen is a solid relief area. According to embodiments of the present invention, hence, the solution may applied from a screen whose relief area is arranged in a preferably regular pattern and has a coverage more than 0% and less than 100%. Preferable coverages comprise at least 20% or at least 30% as lower limits, and less than 75% or less than 60% as upper limits. This may apply to the full width or at least one or at least two of the zones.

Printing, in particular flexographic printing with a screening as described on the print roll, allows adjusting the amount of solution applied to obtain certain target oil pick-up (OPU) values of surfactant in the product, i.e. the resulting hydrophilized nonwoven material. An OPA percentage is determined by extracting the surfactant from a piece of material and determining its amount. The OPU is a measure of how much surfactant is applied to the nonwoven material in a given zone, which the test piece represents. Main determinants for adjusting OPU comprise print roll surface, inter alia screen coverage, concentration of surfactant in the solution and application pressure. In embodiments, the method is configured such that the oil pick-up (OPU) of surfactant in the resulting nonwoven material is between 0,2-3,0 %. Preferred values comprise at least 0,3% or at least 0,4% as lower limits, and less than 1,5% or less than 1,0% as upper limits. OPU can be determined as described further below in the context of the examples. This may apply to the full width or at least one or at least two of the zones.

In an embodiment, the solution of the surfactant comprises, as a solvent, an organic solvent or a mixture of water and an organic solvent. The organic solvent can, for example, be an alcoholic solvent like ethanol. In the case of a mixture, the fraction of the organic solvent in the mixture can be greater 50 wt%, greater 75 wt% or even greater 90 wt%.

Regarding the concentration of the surfactant in the solvent, it can be preferred to have a minimum concentration of at least 5 wt%, preferably at least 15 wt%, and more preferably at least 25 wt%. A maximum concentration is usually a result of certain limitations to viscosity. Depending on surfactant and solvent, maximum concentrations can be around 40 wt%. The indications of wt% given in this paragraph are based on the total weight of the solution,

In embodiments, the solution of the surfactant comprises less than 5wt% and preferably less than 2 wt% of solids, in particular pigments, or other coloring agents. To the contrary, printing inks usually comprise a significant amount of solid pigments or other coloring agents. 15-50 wt% would be a typical value. Additional components in inks comprise binders and additives to disperse the pigments. On the other hand, a surfactant as the surfactants listed above would be detrimental in an ink as the surfactant would be detrimental to ink adhesion on the surface.

A nonwoven material of the invention, as a result of having been made by a method of the invention, has a surface, which is at least in part treated for increased hydrophilicity by having deposited a surfactant thereon by printing. Corresponding to the method, in embodiments, the surface comprises at least two zones having different deposition amounts of surfactant. The at least two deposition amounts may be non-zero, at least one of the zones may have a shape other than machine-directional stripes with a constant width and linear (straight) machine-directional boundary, or both.

Further corresponding to the method, in embodiments, the surfactant is deposited in a preferably regular pattern that covers more than 0% and less than 100% of the zone, preferably between 20-75%, more preferably between 30-60%. The oil pick-up (OPU) of surfactant may be between 0,2-3,0 %, preferably between 0,3-1,5 %, more preferably between 0,4-1,0%. This may apply to the full width or at least one or at least two of the zones.

The nonwoven may have a basis weight of between 5-40 g/m². The nonwoven material may be formed from polyolefin polymers, in particular polypropylene, polyethylene, polyethylene-propylene-copolymers, and mixtures thereof.

### Brief description of the figures

Further details and advantages of the present invention are in the following explained with reference to figures and examples. The figures show:
- Fig. 1:: a schematic side view illustration of kiss roll unit, outside the scope of the invention;
- Fig. 2:: a schematic side view illustration of a flexographic printing unit, as used in embodiments of the invention;
- Fig. 3:: a detailed view of a roll arrangement within the flexographic printing unit of Fig. 2;
- Fig. 4:: examples of screens that can appear on the print sleeve of the unit of Fig. 2-3;
- Fig. 5:: an example of a pattern of hydrophilic areas on a nonwoven material that is obtainable with the method of the invention;
- Fig. 6:: another example of a pattern of hydrophilic areas on a nonwoven material that is obtainable with the method of the invention;
- Fig. 7:: another example of a pattern of hydrophilic areas on a nonwoven material that is obtainable with the method of the invention;
- Fig. 8:: another example of a pattern of hydrophilic areas on a nonwoven material that is obtainable with the method of the invention; and
- Fig. 9:: yet another example of a pattern of hydrophilic areas on a nonwoven material that is obtainable with the method of the invention.

### Flexographic printing versus kiss roll application

For understanding of the present invention, it is important to appreciate the difference between kiss roll application and flexographic printing. Both are methods of applying materials to a surface, but they are different in various ways and are used for different purposes in the prior art.

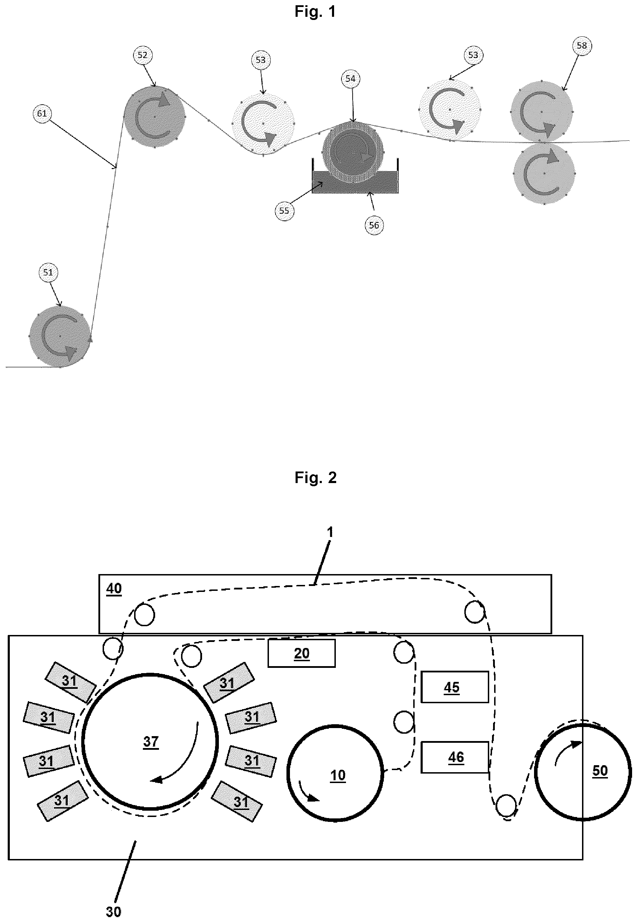

Fig. 1 illustrates a kiss roll application. The material 61, which may be a nonwoven material, travels in machine direction and passes some initial guide rolls 51, 52 before reaching the kiss roll 54. The kiss roll 54 is framed by height-adjustable lay-on rolls 53 that press down the material to create a contact angle on the kiss roll 54. The kiss roll 54 is half way submerged in a bath 56 of liquid solution 55, which may be a solution of a surfactant, and rotates. In the rotation of the kiss roll 54, it picks up solution 55 and deposits it to the material 61. The amount of solution 55 deposited depends on the rotation speed of the kiss roll 54. The level of solution 55 in the bath 56 is kept at the same level by continuously adding a slow flow of solution 55. After leaving the kiss roll 54 and passing the second of the lay-on rolls 53, the material 61 is squeezed between nip rolls 58 to make sure that the solution 55 and dissolved compound, for example surfactant, is spread evenly on the surface of the material 61. After this, the material 61 enters an oven for drying.

The surface of the kiss roll 54 is flat and homogenous. If it is desired in an application to not apply the solution 55 to certain parts of the material 61, plastic flaps may be used to prevent the material 61 from picking up surfactant from the kiss roll 54 by making sure that there is no contact. This allows for application in the form of machine-directional stripes with a constant width and linear (straight) machine-directional boundary.

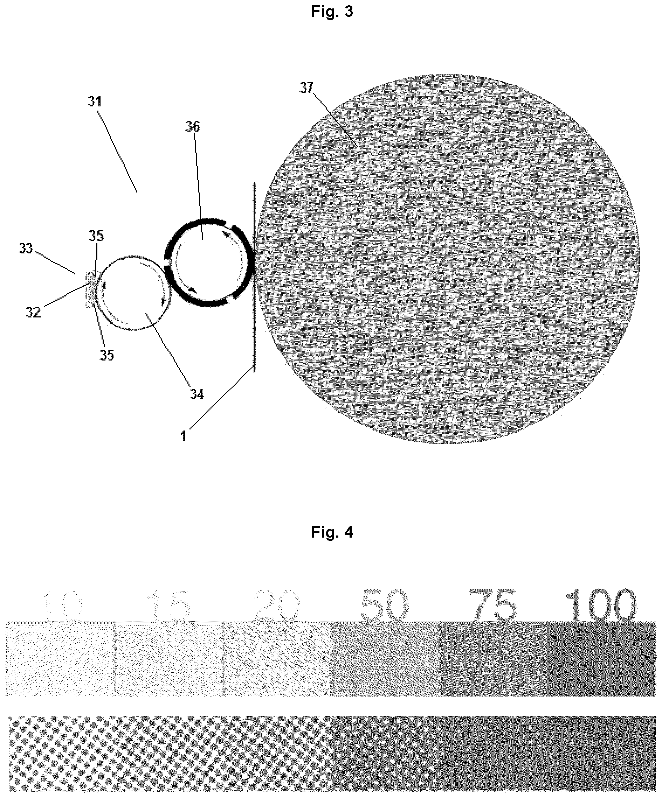

Fig. 2-3 illustrate a flexographic printing application. As shown in Fig. 2, a nonwoven material reservoir comprising an unwinder 10 is followed by a corona treatment station 20, where the surface of the nonwoven material 1, on which, in the invention, the solution comprising the surfactant can be modified. The corona treatment is optional in the context of the invention. The possibly surface-modified nonwoven material 1 then enters a printing unit 30, where multiple roll arrangements 31 for solution transfer, each configured as in Fig. 3 discussed below, are arranged around one common large impression drum 37 for possible multiple step or multiple layer printing. In principle, in the present invention, one arrangement 31 is sufficient, but multiple arrangements can be used to print multiple zones of the nonwoven material surface individually, or carry out layered printing for higher surfactant load on certain zones. In addition, it is possible to combine the surfactant printing of the present invention with regular ink printing in one line. Specifically, some of the arrangements 31 can be configured for regular printing with ink, while one or more other arrangements 31 can be configured to print a surfactant, according to the invention. In case of such combination, it can be preferred that the ink printing is in line before the surfactant printing, at least of the same zones of the nonwoven surface shall be ink printed and surfactant printed, as the surfactant would be detrimental to ink adhesion on the surface of the nonwoven material. The printing unit 30 is followed by a drying unit 40 and product nonwoven material is collected on a rewinder 50. Between the drying unit 40 and the rewinder 50 there can be a vision control and metal detecting unit 45 and a cutting unit 46. They operate for quality control and cutting material longitudinally.

Fig. 3 shows the multiple roll arrangements 31 in detail. The roll arrangement 31 comprises a chambered doctor blade system 33, which is a manifold to deliver the surfactant solution to an anilox roll 34. The solution is added into the bucket and pumped into the reservoir 32, where it is retained by valves, seals and doctor blades 35. The reservoir 32 is kept under pressure by pumping more solution into it than being used. Exit valves on each side of the reservoir control the pressure and lets out excess solution and return it to the bucket. Facing the anilox 34, one doctor blade 35 acts as a retaining blade and holds solution within the reservoir. Another reverse angle doctor blade 35 scraps excess solution from the surface of the anilox roll 34. Specifically, the anilox roll 34 has on its surface tiny engraved cells that enable dosing the solution to be transferred to the subsequent print roll 36. The size of the cells on an anilox roll 34 can vary in volume depending on amount that is intended to be transferred. The surface of the print roll 36 comprises a positive mirrored master of the required image as a 3D relief and transfers solution to the nonwoven material 1. During the transfer, the nonwoven material 1 is sandwiched between the print roll 36 and the rotating impression drum 37 for pressure application.

As can be seen from the above, key differences between kill roll and flexographic printing applications comprise the roll number, the application pressure and the purpose and structure of the kiss roll versus the print roll. Flexographic printing involves multiple rolls (anilox roll 34, the print roll 36, typically comprising a plate cylinder with a print sleeve, and an impression cylinder 37) for metering solution, transferring in in a pattern, and applying pressure. Kiss roll application involves fewer rolls, usually only the kiss roll and possibly a backup roll for simple area coating application. Flexographic printing, over the tiny cells on the anilox roll 34 and the structure of the print roll 36, allows to precisely control the amount of solution that is applied to each portion of the nonwoven substrate 1, while in kiss roll application, emphasis on uniformity and consistency of the coating thickness rather than precision of solution placement.

### Surfactant solution versus ink

Another important difference to be appreciated in the context of the invention is a difference between a solution comprising surfactant on the one hand and an ink on the other hand. A printing ink is primarily made from four components, which are pigments, binders, additives and solvents (water or alcohol). Pigments and possible also further components are solids that are dispersed in the solvent. In a printing ink, the solid contents is typically between 15% to 50%. The purpose of the binders and additives in the ink is to provide sufficient transport of the pigment from the ink, through the various rolls in a print unit and onto the substrate. This is to create a visual appearance on the substrate and provide some performance in terms of color density and adhesion towards, dry, wet and various oily or lotion substances. The solution containing the surfactant, as used in the present invention, to the contrary is mainly made from liquids with no or very small amounts of solids (<5 wt%, more typically <2wt%). The purpose of the surfactant, or surfactant, is to orient the molecules on the substrate surface to be attracted towards water by changing the surface energy.

### Method for measuring OPU

As used herein, the OPU levels are as determined by extracting the oil from the test piece using IPA alcohol. The IPA alcohol evaporates, leaving behind the clean oil. The oil percentage is determined through repeated measurement, i.e. at least two measurements as described below are performed.

A 2 g sample piece of a nonwoven material having a hydrophilic agent printed thereon is prepared and weighted in. The sample weight is V1. A small receptacle, e.g. a small foil tray is then prepared and weighted in. The receptacle weight is V2. The surfactant is then extracted from the sample using isopropanol and the isopropanol comprising the surfactant collected in the receptacle.

The extraction comprises putting the 2 gram nonwoven material sample in a syringe and filling the syringe with 10 ml of isopropanol. When the isopropanol is distributed around the sample, the isopropanol is slowly pushed out from the syringe using a controlled pressure of 50 N on the plunger, which can be adjusted with a torque wrench, and collected in the receptacle. The syringe is then filled with another volume of 10 ml of isopropanol and the process repeated. The receptacle then contains 20 ml of isopropanol that has the surfactant from the sample dissolved therein.

The isopropanol is then fully evaporated by heating and the receptacle allowed to cool down after evaporation is complete. The receptacle, which now comprises the extracted surfactant as a non-evaporated residue, is then again weighted in and the weight recorded as weight V3. The OPU is calculated as OPU = (V3-V2)/V1 × 100%.

The OPU percentage is relative to the nonwoven material weight V1, but within the boundaries of standard basis weights of such materials used in adsorbent hygiene products of, e.g., 5 to 40 g/m², the ranges indicated herein are applicable independently from the basis weight nonetheless because a higher basis weight nonwoven requires more surfactant for allowing strike through of bodily fluids.

### Exemplary embodiments

The print roll 36 (Fig 3) of the flexographic printing unit 31 in the present application is typically made from a cylinder and print sleeve mounted thereon. The print sleeve can be made from a synthetic material, which has a given hardness that accommodate the nonwoven material it will be printing on. The surface design can be made on it by laser engraving where all nondesign areas are removed, to a certain depth (relief height) by the laser and only the mirrored design is left back on the sleeve. Additionally, the surface remaining representing the design can be made with various screening to make the area more or less concentrated.

Examples of various ways of screening shown in Fig. 4. Specifically, Fig. 4 shows various screens that can appear on the print sleeve. The top bar illustrates various percentage of screening. The bottom bar illustrates, up close, how above screening percentages are created by having tiny dots of relief peaks as surface and areas where the peaks are removed i.e. 50% screen is an equal mix of peaks and open area and 100% screen is a solid relief area.

The print sleeve can be designed to contain any geometric or odd shape, which may contains one or several different screenings. The shapes can be repeated with the same distance in the machine direction or cross-machine direction, or randomly positioned. By applying surfactant containing solution to the print sleeve, in a choice as described above, the print sleeve will then deposit the solution onto the nonwoven material, thereby providing a result that is different from a result that would be obtained using a kiss roll application. Specifically, hydrophilic areas do not have to be continuous or in machine-directional stripes, but can have any odd shape, and the shapes can have various OPU levels between, for example, 0,2 and 3,0% for nonwoven materials having a basis weight of between 5 and 40 g/m².

In the following, examples of patterns of hydrophilic areas on nonwoven materials that are treated in a method of the invention are described.

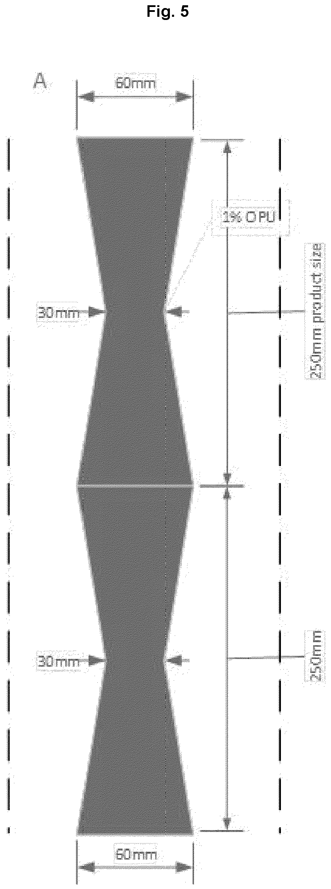

Fig. 5 shows a continuous machine directional printed hydrophilic zone having a varying width. The width variance and length is made in register to correspond to the length of a final size of an absorbent hygiene products (AHP). The hydrophilic zones in this example are made with one OPU % level, which can be, for example, any level between 0,2% and 3%. The dotted lines in Fig. 5 indicate where the material will be cut in width to be used for the AHP.

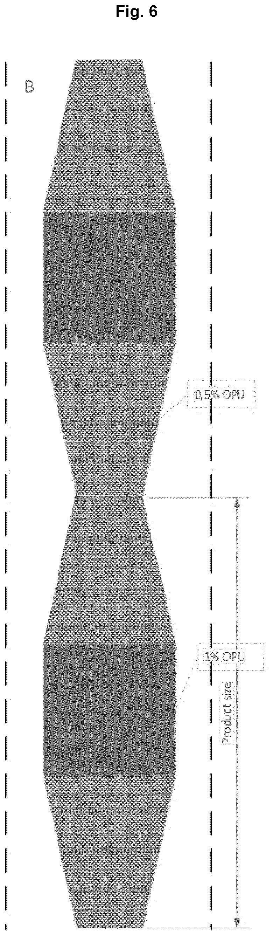

Fig. 6 shows a variant of Fig. 5 where the hydrophilic zone is made with two OPU % levels, one high level (e.g., OPU 1%) in the middle of the product and another (e.g., OPU 0,5%) on the front and back of the AHP. It is understood that the zones can be made with any other OPU % between, e.g., 0,2% and 3%.

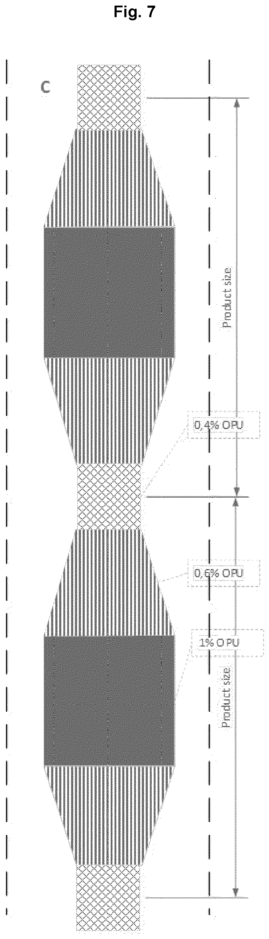

Fig. 7 shows another variant of the pattern of Fig. 5-6, where the hydrophilic zone is differently shaped and comprises three zones of different OPU % levels, namely one high level zone (e.g., OPU 1%) in the middle of the product, a second zone (e.g., OPU 0,6%) on either side of middle zone, and a third zone (e.g., OPU 0,4%) on either end of the second zone. It is understood that the zones can be made with any other OPU % between, e.g., 0,2% and 3%. In an alternative, it would be an option to discard the third zone and have it surfactant free.

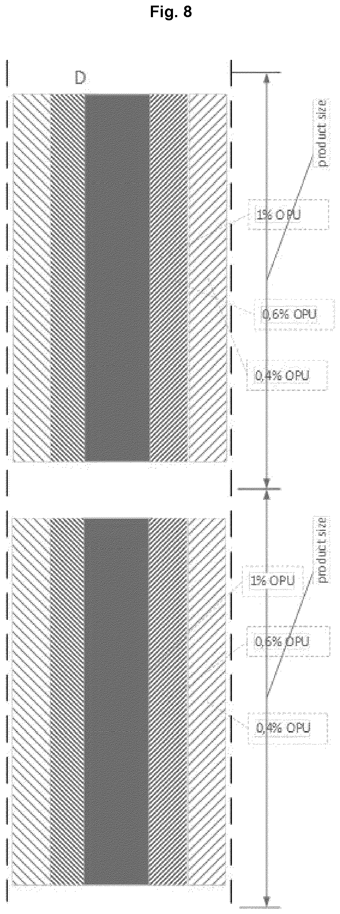

Fig. 8 shows yet another alternative example of a pattern of hydrophilic areas on a nonwoven material that is obtainable with the method of the invention. The pattern of Fig. 8 comprises several registered straight machine directional printed hydrophilic zones aligned next to each other. The length of the zones is made in register to correspond to the length of the final size of the AHP. The hydrophilic zones are made again with three OPU % levels, one high level zone (e.g., OPU 1%) in the center of the product, second zones (e.g., OPU 0,6%) on either side of center zone, and third zones (e.g., OPU 0,4%) on either side of the second zones. It is again understood that the zones can be made with any other OPU % between, e.g., 0,2% and 3%. After a gap width no surfactant in the machine direction the zones are repeated. Again, the dotted lines indicate where the material will be cut in width to be used for the AHP. Alternatives may comprise having all zones continuous without gaps, or discarding a zone to have a surfactant free zone in the AHP, or both.

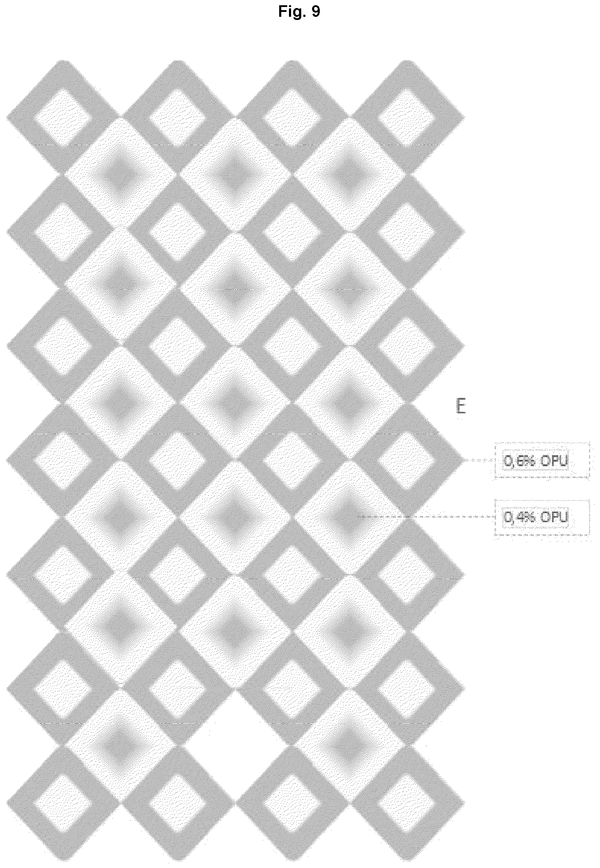

Fig. 9 shows yet another alternative example of a pattern of hydrophilic areas on a nonwoven material that is obtainable with the method of the invention. Here, the pattern is a random hydrophilic pattern with two OPU levels. White areas have no surfactant printed. The zones can be made with any OPU % between, e.g., 0,2% and 3%.

The examples demonstrate the benefit of the present invention. Specifically, as compared to prior art kiss roll application of surfactant, the method of the invention allows using less surfactant and concentrating it where it is needed. By having more surfactant in the center of a nonwoven material piece in an adsorbent hygiene product than on the sides, side leakage can be avoided. By having more surfactant in the center of a nonwoven material piece in an adsorbent hygiene product than in front and back, flow of fluids more effectively be lead to the core of an adsorbent hygiene product. By mixing up high and low surfactant areas, liquid can be distributed more evenly into the core. In more general terms, having surfactant zones having a particular shape and surfactant load on the nonwoven materials, the flow of bodily fluids into the core of an absorbent hygiene product can be controlled better.

## Claims

1. A method for producing a nonwoven material, whose surface is at least in part treated for increased hydrophilicity by depositing a solution of a surfactant on the surface,
**characterized in that**
the solution of surfactant is applied to the surface by printing.

2. The method according to claim 1, wherein the printing is flexographic printing, using an anilox roll and a print roll to transfer the solution of surfactant from a source to the surface of the nonwoven material while the nonwoven material translates through a nip between the print roll and an impression drum.

3. The method according to any preceding claim, wherein the application is such that at least two different amounts of the solution are applied to at least two different zones of the surface.

4. The method according to claim 3, wherein at least one of the zones has a shape other than machine-directional stripes with a constant width and linear boundary.

5. The method according to claim 3 or 4, wherein the at least two different amounts of the solution that are applied to the at least two different zones of the surface are at least two different non-zero amounts.

6. The method according to any preceding claim, wherein the solution is applied from a screen whose relief area is arranged in a preferably regular pattern and has a coverage more than 0% and less than 100%, preferably between 20-75%, more preferably between 30-60%.

7. The method according to any preceding claim, wherein the solution is applied in an amount such that the oil pick-up (OPU) of surfactant in the resulting nonwoven material, when determined according to the specification, is between 0,2-3,0 %, preferably between 0,3-1,5 %, more preferably between 0,4-1,0%.

8. The method according to any preceding claim, wherein the solution of the surfactant comprises, as a solvent, an organic solvent or a mixture of water and an organic solvent.

9. The method according to any preceding claim, wherein a concentration of the surfactant in the solution of surfactant is at least 15 wt%, preferably at least 25 wt%, based on the total weight of the solution.

10. The method according to any preceding claim, wherein the solution of the surfactant comprises less than 2 wt% of solids.

11. A nonwoven material made by a method of any preceding claim, wherein the surface of the nonwoven material is at least in part treated for increased hydrophilicity by having deposited a surfactant thereon.

12. The nonwoven material of claim 11, wherein there are at least two zones on the surface comprising different deposition amounts of surfactant.

13. The nonwoven material of claim 12, wherein the at least two deposition amounts are non-zero, or at least one of the zones has a shape other than machine-directional stripes with a constant width and linear boundary, or both.

14. The nonwoven material of any one of claims 11-13, wherein the surfactant is deposited in a preferably regular pattern that covers more than 0% and less than 100% of the zone, preferably between 20-75%, more preferably between 30-60%.

15. The nonwoven material of any one of claims 11-14, wherein the oil pick-up (OPU) of surfactant, when determined according to the specification, is between 0,2-3,0 %, preferably between 0,3-1,5 %, more preferably between 0,4-1,0%.
